# EUROPEAN PATENT APPLICATION

(11) **EP 3 456 243 A1**
(43) Date of publication of application: **20.03.2019**
(21) Application number: 17190995.5
(22) Date of filing: 14.09.2017
(51) Int. Cl.: A61B 5/00, A61B 6/00, A61B 5/02, G06F 19/00, G06T 5/50, G06T 7/00, A61B 90/00

(54) **IMPROVED VESSEL GEOMETRY AND ADDITIONAL BOUNDARY CONDITIONS FOR HEMODYNAMIC FFR/IFR SIMULATIONS FROM INTRAVASCULAR IMAGING**

(71) Applicant: Koninklijke Philips N.V., 5656 AE Eindhoven (NL)
(72) Inventor: HAASE, Christian, 5656 AE Eindhoven (NL); GRASS, Michael, 5656 AE Eindhoven (NL); VAN LAVIEREN, Martijn, 5656 AE Eindhoven (NL)
(74) Representative: de Haan, Poul Erik

(57) **Abstract**

An apparatus for modeling coronary physiology and a corresponding method are provided in which at least one diagnostic image and intravascular imaging data are co-registered and used in combination to retrieve more accurate, patient-specific vessel parameters to be used in the modeling of the vessel geometry and the fluid dynamics of the blood flow.

## Description

### FIELD OF THE INVENTION

The present invention relates to an apparatus for modeling the coronary physiology of a patient, a corresponding method and a respective computer program. In particular, the present invention relates to an improved modeling of the vessels in the coronary physiology using a co-registration of non-invasively obtained diagnostic images with intravascular image data that has been obtained in-situ in the vessels of the coronary physiology.

### BACKGROUND OF THE INVENTION

Functional stenosis in coronary arteries is typically graded using Fractional Flow Reserve (FFR) or Instantaneous Wave-Free Ratio (iFR). Both, FFR as well as iFR, are a measure for the pressure drop of the blood along the stenosis, i.e. are determined as the ratio of the pressure distal the stenosis (P_{d}) to the pressure in the aorta (Pₐ).

To that end, FFR measurements have to be performed during maximal blood flow, i.e. under hyperemia, which may cause discomfort in the patient. In contrast, iFR measurements are performed at rest during a specific period in diastole, thereby avoiding the necessity to induce hyperemia in the patient. By foregoing this necessity of hyperemia, the comfort of the patient is greatly improved.

In the past, the determination of FFR and/or iFR was done invasively by performing a pullback of a pressure wire through a vessel of the patient in which the stenosis is suspected and by determining the pressure for a plurality of locations along said vessel. In recent years, however, approaches have been made to determine FFR/ iFR values non-invasively. To that end, the so-called "virtual" FFR/iFR has been developed. Hereby, the fluid dynamics in the coronary arteries of a patient are simulated on the basis of a physiological model derived from non-invasively obtained diagnostic image data of the patient's heart (or a part thereof), such as coronary angiography data. The physiological model hereby comprises a geometric model representing the vessel or vessel tree under examination and a fluid dynamics model representing the blood flow through said vessel or vessel tree. By means of such a modeling, an FFR/iFR value may be estimated and a decision may be made on the basis of this estimated FFR/iFR value which medical procedures are necessary for the patient.

So far, approaches in modeling coronary physiology to estimate the FFR/iFR value have been focused on using 3D imaging data comprising a plurality of images in combination with generalized boundary conditions to provide a physiological model representing vessel geometry and fluid dynamics of the vessels of a patient. In recent years, the necessity to provide more accurate simulations on the basis of imaging methods that require less radiation has become more and more important.

In that respect, WO 2016/001017 discloses an apparatus and a method for improving the accuracy of non-invasive determination of the FFR value based on a representation of the coronary artery system of a patient and respective patient-specific boundary conditions derived from projection data obtained for the patient. Hereby, the projection data is generated by a detector over time while irradiating the patient with radiation generated by a radiation source. Accordingly, this approach requires exposing the patient to a certain amount of radiation necessary to obtain the projection data.

### SUMMARY OF THE INVENTION

In order to reduce the amount of radiation used for obtaining the diagnostic images, the modeling of the coronary physiology may be performed on the basis of a single 2D diagnostic image obtained using X-ray angiography. A 2D image does not contain any information about the coronary artery in a third dimension. Thus, the vessel geometry may only be derived accurately in two dimensions with the third dimension having to be estimated. This is a valid approach for vessels which are projected with low foreshortening, which corresponds to a vessel segment direction perpendicular to the ray direction of the projection, while it has limitations for vessels with high foreshortening. Also, the estimation of asymmetric cross sectional vessel area may be more difficult. Thus, since accurate knowledge of the vessel geometry is of critical importance for the estimation of the FFR/iFR value, employing 2D image-based modeling may not lead to results as accurate as those employing an approach based on 3D imaging data.

As an additional factor, the fluid dynamic of the blood flow through the vessels is influenced by factors such as vessel wall composition, vessel impedance, vessel wall elasticity or the like. Accordingly, in order to accurately perform virtual FFR/iFR, it is necessary to take account for these factors using particular boundary conditions in the fluid dynamic simulation. Hereby, these boundary conditions are generally based on empirical values rather than being determined for each patient individually. This introduces a further source of inaccuracy into the physiological simulations.

It is therefore an object of the present invention to provide an improved apparatus and a corresponding method for modeling coronary physiology of a patient.

It is a further object of the invention to provide an apparatus for modeling the coronary physiology of a patient with high accuracy which allows for a patient-specific simulation of the fluid dynamics of the patient's blood flow. Even more particularly, it is an object of the present invention to provide an apparatus for modeling of the coronary physiology on the basis of one or more diagnostic images with which virtual FFR/iFR values may be determined with high accuracy.

Therefore, an apparatus for modeling coronary physiology is provided which comprises an input unit configured to receive at least one diagnostic image of a vasculature of a patient and intravascular image data obtained in-situ from a vessel of the vasculature. The apparatus further comprises a modeling unit configured to generate a physiological model of the patient on the basis of the at least one diagnostic image, whereby the physiological model comprises a geometric model of the vessel, and an extraction unit configured to extract at least one vessel parameter of the vessel from the intravascular image data. The apparatus further comprises an adaption unit configured to adapt the physiological model on the basis of the at least one vessel parameter.

Hereby, at least one diagnostic image is obtained from a patient. The term diagnostic image may hereby refer to an image representing the coronary arteries. More particularly, the diagnostic image may represent a vasculature including a vessel of interest of the heart. In this context, the term vasculature may refer to a vessel tree or a single vessel. The term vasculature may particularly refer to a vessel segment of the vessel of interest, for which a modeling is performed.

In some embodiments, the diagnostic image may be obtained by a non-invasive diagnostic imaging modality, such as computed tomography (CT) or magnetic resonance imaging. In one particular embodiment, a single diagnostic 2D X-ray angiography image may be obtained.

The non-invasive diagnostic imaging modality may particularly be gated. Hereby, the gated non-invasive diagnostic imaging modalities may typically employ a gated reconstruction, in which the acquisition of the diagnostic images is performed in parallel with acquisition of data providing information over the cardiac cycle, such as electrocardiogram (ECG) or photoplethysmographic (PPG) data. This data may hereby be used to gate the image acquisition and the reconstruction by means of respectively selected phase points of the cardiac cycle.

In an embodiment, a physiological model may be derived from the at least one diagnostic image to simulate the coronary physiology of the patient. The term physiological model may hereby refer to a model of the coronary physiology of the patient.

Hereby, a modeling unit is provided which generates the physiological model from the diagnostic image. In order to do so, the modeling unit may segment the vessel of interest into a plurality of segments. On the basis of this segmentation, the physiological model of the vessel of interest may be generated, which comprises a geometric model of the vessel of interest, i.e. a geometric representation of the vessel of interest. This geometric model may be a two- or a three-dimensional model.

The physiological model may further comprise a fluid dynamics model representing the blood flow through the vessel of interest. The fluid dynamics model may typically be integrated with the geometric model, insofar as it represents the fluid dynamics of the blood at each point of the geometric model.

The fluid dynamics model is generated by performing calculations that simulate the interaction of the blood with the vessel wall, i.e. the inner surface of the vessel through which the blood is flowing. These interactions are hereby defined by certain boundary conditions that take account of the properties of the vessel, such as vessel wall composition, vessel wall elasticity and vessel impedance, bifurcations in the vessel as well as blood properties, such as blood viscosity.

When generating the fluid dynamics model, typically, generalized boundary conditions may be used. In this context, the term generalized may refer to the same boundary conditions being used for all patients or to boundary conditions being used for particular patient groups (distinguished by age, gender, physiological condition or the like).

Further to the diagnostic image, intravascular image data is also received at the input unit. The term intravascular image data particularly refers to image data that has been obtained in-situ, i.e. from inside the vessel, using an intravascular imaging modality. This may typically be performed by introducing a catheter including an imaging device into the vessel of interest. The imaging device may then collect image data from inside the vessel at one or more particular intravascular imaging positions.

The intravascular image data may comprise a single intravascular image or a time series of intravascular images collected for the same intravascular imaging position over time. Additionally or alternatively, the intravascular image data may also be collected by slowly pulling back the catheter through the vessel and by obtaining a plurality of intravascular images at a plurality of intravascular imaging positions.

The intravascular image data may particularly be collected in the same session as the at least one diagnostic image. In an embodiment, the intravascular image data may be obtained concurrently with the diagnostic image. Alternatively or additionally, the intravascular image data may be obtained before or after acquisition of the diagnostic image. In a further embodiment, the intravascular image data may be collected in a different session than the diagnostic image data.

The intravascular imaging data is provided to an extraction unit which extracts at least one vessel parameter of the vessel of interest from the intravascular imaging data. The at least one extracted vessel parameter is then provided to an adaption unit for adapting the physiological model.

Hereby, the adapting on the basis of the at least one vessel parameter refers to an updating of the physiological model using the information from the vessel parameters derived from the intravascular image data.

To that end, the intravascular imaging modality providing the intravascular image data may particularly be used to provide additional information about the patient that may not be derived or not be derived accurately from the at least one diagnostic image. More particularly, the additional information that may be obtained using the intravascular imaging modality may relate to geometric and/or hemodynamic parameters derivable from the intravascular image data.

In this context, the term geometric parameter may refer to any parameter that is representative of the geometry of the vessel of interest. In some embodiments, the geometric parameter may particularly refer to the cross sectional vessel lumen of the vessel of interest at a plurality of positions along the length of the vessel of interest. Further, the geometric parameter may also refer to the inner shape of the vessel or similar geometric measures. Alternatively or additionally, the additional information may refer to a hemodynamic parameter. In this context, hemodynamic parameters may for example refer the vessel wall elasticity, the vessel wall friction, the vessel impedance or the blood viscosity or the like.

According to an example, additional information may particularly refer to information about plaque location, composition, or associated rupture risk. More specifically, a segment of the vessel of interest of a patient may exhibit positive wall remodeling caused by plaque inside the vessel wall. Such positive wall remodeling may not be visible in the diagnostic image, which may lead to incorrect conclusions about the health state of the patient. However, the positive wall remodeling will be visible in the intravascular image data and may be used for adapting the physiological model to more accurately represent the vessel of interest.

Alternatively or additionally, the additional information may refer to information about bifurcations from the vessel of interest. Since the intravascular image data provides an image of the inside of the vessel, small bifurcations may be visible in the intravascular images of the intravascular imaging data. More particularly, the position, size and/or direction of the bifurcations may be derived from the intravascular images. Since bifurcations represent outflows from the vessel of interest, this information may be used to modify the boundary conditions of the fluid dynamics model with respect to the outflow from the vessel.

As such, the adapting of the physiological model may in particular refer to an adjustment of the geometric model of the vessel of interest in accordance with the geometric parameters derived from the one or more intravascular images. Even more particularly, the adapting of the physiological model may refer to an adjustment of the geometric model of the vessel of interest in which a geometric parameter that has been approximated from the diagnostic image is replaced by an invasively measured geometric parameter that has been extracted from the intravascular image data.

Further, the adapting of the physiological model may refer to an adjustment of the fluid dynamics model representing the blood flow through the vessel of interest. Hereby, the vessel parameters extracted from the intravascular image data are hemodynamic parameters that are specific to the respective patient. These patient-specific hemodynamic parameters may be used to determine patient-specific boundary conditions to the fluid dynamics model. Using the patient-specific boundary conditions, the calculation of the fluid dynamic model may be performed more accurately.

In some embodiments, the adaption unit may be provided as a processing unit adapted to perform an adaption algorithm, in which the geometric and/or hemodynamic parameters are used to adjust the geometric and/or the fluid dynamics model, respectively. This adaption algorithm may be implemented as a machine learning algorithm. More specifically, the machine learning algorithm may particularly be used for adapting the boundary conditions for the fluid dynamics model based on the hemodynamic parameters derived from the intravascular imaging data.

According to an embodiment, the at least one diagnostic image is obtained using X-ray angiography. X-ray angiography is a diagnostic imaging technique particularly well-suited to visualize blood vessels in the (human) body. X-ray angiography is typically performed by injecting a contrast agent into the blood vessels and subsequently irradiating the body part with the contrast agent-filled blood vessels with X-ray radiation to obtain a two-dimensional image in which the contrast agent-filled blood vessels are clearly visible.

In the context of the invention, the diagnostic image may thus particularly be an X-ray angiography image of the coronary arteries of the patient comprising at least one vessel of interest. More particularly, the at least one diagnostic image from which the physiological model is generated may be a single 2D X-ray angiography image of a vessel of interest. In this case, the physiological model may comprise a two-dimensional geometric model in which a third dimension is approximated, e.g. by assuming a circular shape of the vessel.

In order to obtain a sufficiently well-resolved physiological model, the X-ray angiography image should hereby have sufficient contrast agent filling. Furthermore, the degree of foreshortening and overlap in the X-ray angiography image should be sufficiently low. This increases the quality of the image and simplifies the generation of the physiological model.
In a further embodiment, the intravascular image data is obtained using intravascular ultrasound (IVUS) and/or optical coherence tomography (OCT).

Hereby, the IVUS and/or OCT measurements may particularly be performed using gated IVUS and/or OCT to perform a phase matching with the phase used for acquiring the diagnostic images. More particularly, a phase matching with the phase of the X-ray angiography may be performed.

Intravascular ultrasound is generally considered a well-suited intravascular imaging method to obtain information about the coronary arteries. In particular, it enables to visualize not only the lumen but also the atheroma in the vessel walls. As such, it is typically used to determine the amount of plaque in the blood vessels.

In intravascular ultrasound, a catheter is used which is equipped with a miniature ultrasound probe attached to its distal end, while respective ultrasound equipment is attached to the proximal end. The catheter is then introduced into the blood vessel, thereby allowing the application of ultrasound inside the blood vessel. This allows obtaining a respective intravascular ultrasound image of the inside of the vessel.

In some embodiments, a single intravascular ultrasound image or a time series of intravascular ultrasound images of the vessel of interest may be obtained for a particular intravascular imaging position. Further, a plurality of intravascular ultrasound images may be obtained at a plurality of intravascular imaging positions along the vessel of interest. Particularly, the plurality of intravascular ultrasound images may be obtained at the plurality of intravascular imaging positions along the vessel by slowly pulling the catheter through said vessel.

By means of this pullback acquisition, it is possible to obtain the respective geometric parameters of the vessel at several intravascular positions along a vessel segment of a particular length.

Optical coherence tomography is an established diagnostic imaging technique which is likewise considered suitable for visualization of the coronary physiology, in particular respective vessel(s) of interest. The benefit of optical coherence tomography is its enhanced resolution compared to other modalities. Hereby, a single optical coherence tomography image, a time series of optical coherence tomography images or a plurality of optical coherence tomography images at a plurality of intravascular imaging positions along the vessel of interest may be collected.

In order to perform the optical coherence tomography measurements, a fiber-optics catheter is introduced into the blood vessel of interest. Subsequently, a contrast agent and/or a saline solution is injected into said vessel of interest.

Hereby, the fiber-optics catheter is used to introduce light, typically in the near-infrared, into the vessel. The light reflected in the vessel is then detected in order to derive an optical tomography image. OCT is a fast and very high resolution imaging modality. Due to this high resolution, the information obtained about the vessel (in particular the vessel wall) is very accurate. Thus, by means of this information, the quality of the physiological model of the vessel of interest may be greatly improved. Furthermore, OCT may be performed with high temporal resolution, thereby allowing to obtain a temporally resolved physiological model with good temporal resolution. Therefore, OCT may be considered well-suited for time-series measurements during the cardiac cycle.

In some embodiments, the obtaining of the intravascular imaging data using IVUS and/or OCT measurements may also comprise measuring information about the blood flow through the vessel of interest, such as performing bolus passage measurements. This additional information about the blood flow may then be provided to the adaption unit and used for adapting the fluid dynamics model by adjusting the boundary conditions for the blood flow in accordance with the measurements.

According to an even further embodiment, the intravascular image data comprises a time-series of intravascular images obtained during a cardiac cycle of the patient.

In this context, the time-series of intravascular images may particularly be a time-series of optical coherence tomography images that may be obtained during the cardiac cycle. Hereby the light introduced by the fiber-optics catheters is reflected differently, depending on the changes in the vessel wall geometry during the cardiac cycle, due to movement of the vessel wall. This movement of the vessel wall may particularly provide an information about the vessel wall elasticity, but likewise about further vessel wall properties, for example the vessel wall composition.

According to an even further embodiment, the generating of the physiological model comprises a segmenting of the vessel of the vasculature, whereby the geometric model is generated for at least one segment of the vessel.

In some embodiments, the generating of the physiological model may comprise a segmentation of the imaged vessel of interest into respective vessel segments. On the basis of these segments, the vessel geometry may be modeled using the geometric model. The geometric model may thus provide an estimated representation of the shape of the vessel for the particular vessel segments.

According to a further embodiment, the intravascular image data comprises a plurality of intravascular images. Each one of the plurality of intravascular images has been obtained at one of a plurality of different intravascular imaging positions along the vessel of the vasculature. The input unit is further configured to receive tracking information for correlating each of the plurality of intravascular imaging positions to a respective vessel position in the geometric model.

In this context, the term intravascular imaging position refers to a position inside the vessel of interest at which a particular intravascular image comprised in the intravascular image data has been obtained. Hereby, a plurality of intravascular images may be obtained by performing a pullback acquisition, i.e. by slowly pulling the catheter through the vessel of interest. For a particular segment, each one of the plurality of intravascular images may then be obtained at a particular position which is defined as the intravascular imaging position that is correlated to the respective intravascular image.

Hereby, the catheter position may be tracked during the acquisition of the intravascular image data. This may particularly be done using a fluoroscopic recording of the catheter position. Alternatively, the tracking may be performed using electromagnetic or fiber-optic shape sensing based catheter tracking. Likewise, the tracking may be performed using an image based registration method, i.e. by regarding the diagnostic image and/or the intravascular image and by determining the catheter position using the information that may be derived from said images.

Tracking the catheter position allows for determining the intravascular imaging position at which a respective intravascular image has been obtained during pullback of the catheter. The tracking information may thus be provided along with the intravascular image data to ease the correlation of each of a plurality of intravascular imaging positions to each of a plurality of vessel positions in the geometric model.

In accordance with a further embodiment, the extracting of at least one vessel parameter from the intravascular image data therefore comprises an extraction of a geometric parameter from each one of the plurality of intravascular images obtained at each one of the plurality of the different intravascular imaging positions. Further, the adaptation of the physiological model comprises an adaptation of the geometric model of the vessel at the respective vessel position.

Hereby, the term extract means that a certain parameter is derived from the intravascular image data by means of an image processing procedure, an image analysis procedure or the like. Typically, the geometric parameters may be directly derived when analyzing the one or more intravascular images comprised in the intravascular imaging data. In case the intravascular imaging has been performed by performing a pullback of the catheter through the vessel the geometric parameters may hereby particularly be retrieved for a plurality of intravascular imaging positions.

As discussed, the provision of tracking information enables a correlation of the plurality of intravascular imaging positions in the vessel to a respective plurality of vessel position in the geometric model. This, in turn, allows to adapt the geometric parameters extracted from the intravascular images for a particular intravascular imaging position at the correct vessel position in the geometric model. More particularly, the systematic adapting of the geometric model may comprise an adjustment of the geometric model by replacing an approximated geometric parameter in the geometric model at the particular vessel position with the geometric parameter that has been extracted from the intravascular image obtained at the intravascular imaging position correlated to the vessel position.

As an example, the adapting of the geometric model may comprise a replacing of each of a plurality of approximated cross sectional vessel lumen at their respective vessel positions by a plurality of actual cross sectional vessel lumen as extracted from the respective intravascular images obtained at the particular positions. Hereby, a local lumen smoothing at each vessel position for which the cross sectional vessel lumen has been replaced may be applied in order to provide a smooth physiological model. This lumen smoothing may particularly be performed using an interpolation method, such as a linear or a spline-based interpolation. Likewise, the smoothing may be performed using a Fourier interpolation or an interpolation based on a shape-model.

Further, the intravascular imaging position at which the pullback acquisition has been started, i.e. the first intravascular imaging position for which an intravascular image was obtained, as well as the intravascular imaging position at which the pullback acquisition has ended, i.e. the last intravascular imaging position for which an intravascular image was obtained, should particularly be correlated to a respective first and last vessel position in the geometric model. These first and last vessel positions in the geometric model may be used as cut-off positions, such that the length of the geometric model of the vessel is adapted to the pullback length of the catheter pullback.

By means of adapting the length of the geometric model accordingly, it may be ensured that the geometric parameters, such as the cross sectional vessel lumen, may be adjusted in accordance with the additional information obtained from the intravascular imaging data along the entire length of the geometric model.

This adaption of the geometric model according to the measured cross sectional vessel lumen may further improve the determination of the fluid dynamics for the blood flow through the physiological model. More particularly, since the cross sectional vessel lumen is known and since the blood flow through said cross sectional vessel lumen is closely related to the vessel size, accurate knowledge of the local cross sectional vessel lumen allows to estimate the healthy vessel size more accurately. The healthy vessel size, however, is an important factor to determine the boundary conditions for the fluid dynamics model. Thus, a more accurate geometric representation of the vessel of interest may also result in a better fluid dynamics model for the blood flow through said vessel of interest. As a result, the physiological model has an improved accuracy with respect to the geometry of the vessel and with respect to the fluid dynamics of the blood flow through said vessel.

According to a further embodiment, the apparatus further comprises a display unit configured to display a graphical representation of the physiological model. The adapting of the physiological model is performed in real-time during obtaining of the intravascular image data and the display unit is further configured to update the graphical representation based on the adapting of the physiological model.

The apparatus may further comprise a display unit configured to obtain and display a graphical representation of the physiological model. The display unit may particularly be a computer screen, such as a touch screen or the like.

The adaption unit may use the at least one vessel parameter to adapt the physiological model in real-time during acquisition of the intravascular image data and the display unit may subsequently update the graphical representation of the physiological model in accordance with the adaption performed by the adaption unit.

In this context, the term real-time refers to a situation where the intravascular imaging data is acquired during the modeling, in particular using a pullback acquisition. For each intravascular image obtained at a particular intravascular imaging position, the adaption unit may directly adapt the physiological model and, subsequently, provide the adapted physiological model to the display unit. This adaption may particularly encompass an adapting of the fluid dynamics model based on the at least one vessel parameter. Alternatively or additionally, it may also refer to an adapting of the geometric model.

The display unit may, upon receipt of the adapted model, update the graphical representation to reflect the changes that have occurred in the physiological model due to the adaption. This direct adaption and display updating process may hereby be performed for each new intravascular image received from the intravascular imaging modality or may be performed for a particular image or time interval during intravascular imaging, for example upon receipt of every five intravascular images or every second, every five, ten, fifteen or the like seconds.

This frequent updating of the graphical representation of the physiological model allows a user to more quickly obtain an accurate understanding of the imaged vasculature, in particular for the parts of the physiological model that have already been updated. Furthermore, it allows a user to derive, from the divergence between the initial and the updated graphical representations, the accuracy of the initial physiological model. This may significantly shorten the time the user needs to assess the coronary physiology of the patient.

According to an embodiment, the physiological model further comprises a fluid dynamics model for a blood flow through the geometric model of the vessel. The extracting of at least one vessel parameter from the intravascular image data comprises an extracting of at least one hemodynamic parameter, whereby the adapting of the physiological model comprises an adapting of the fluid dynamics model on the basis of the at least one hemodynamic parameter. The adapting of the physiological model comprises adjusting at least one patient-specific boundary condition for the fluid dynamics model on the basis of the at least one hemodynamic parameter.

In this context, the fluid dynamics model refers to a model of the blood flow through the vessel of interest. This fluid dynamics model is generated by simulating the interaction of the blood with the vessel wall. Since the blood is a fluid and the vessel walls may be considered surfaces with which the fluid interacts, the interaction of the blood with the vessel walls may most accurately be defined by respective boundary conditions that take account of the properties of the vessel wall and the blood interacting with it. These properties may include vessel wall composition, vessel wall elasticity and vessel impedance, bifurcations in the vessel as well as blood viscosity. In the context of the invention, the initial generation of the fluid dynamics model is hereby performed using generalized boundary conditions with the boundary conditions being personalized, i.e. with patient-specific boundary conditions being defined during adaption of the physiological model.

According to an embodiment, the fluid dynamics model may refer to a lumped parameter fluid dynamics models. To that end, the term lumped parameter model refers to a model in which the fluid dynamics of the vessels are approximated by a topology of discrete entities. Hereby, a vasculature, such as a vessel tree, may be represented by a topology of resistor elements each having a particular resistance with the representation of the vessel tree being terminated by respective ground elements. These lumped parameter models reduce the number of dimensions compared to other approaches such as Navier-Stokes or the like. Accordingly, using a lumped parameter model may allow for a simplified calculation of the fluid dynamics inside the vessels and may ultimately result in reduced processing time. The employing of such a lumped parameter model is described for example in international application WO 2016/087396.

According to the above embodiment, the at least one vessel parameter may also be a hemodynamic parameter, i.e. a parameter related to the fluid dynamics of the blood flow inside the vessel. These hemodynamic parameters may be determined for each patient individually. Hence, they allow for replacing the generalized boundary conditions in the fluid dynamics model by patient-specific boundary conditions.

Herein below, a few examples for hemodynamic parameters are provided. Further hemodynamic parameters may be apparent to those skilled in the art.

The intravascular imaging data may comprise so-called speckles which have to be de-correlated to be processed. The de-correlation time of these speckles may be used as an indicator for the local flow velocity for the positions in the vessel. The local flow velocity may then be used to derive a local flow profile and, in particular, the flow distribution including regions with turbulent flow that are specific to the respective patient. The thus derived flow profile may then be used to adapt the fluid dynamics model for the particular patient accordingly.

Further, depending on the measurement, the speckles may have a particular speckle concentration. This speckle concentration may be a measure of blood viscosity for a particular patient. Thus, the blood viscosity for a particular patient may be derived from this data. The respective generalized boundary condition relating to blood viscosity in the fluid dynamics model may then be adapted to a patient-specific boundary condition based on the blood viscosity extracted from the intravascular image data.

In a further example, the amount of saline or contrast agent required to obtain a clear image using intravascular imaging may be used as an indicator for the boundary conditions describing the blood flow in the particular patient. This is particularly so since the saline or contrast agent has to replace the blood inside the vessel. Thus, the amount of saline or contrast agent relates to the total blood volume that has been replaced.

Further, the image quality may be tracked during intravascular imaging. To this end, the change of the image quality with time may be used as an indicator to estimate the response of the coronary circulation of that patient to the lack of oxygen, i.e. the auto regulation response of the heart of the patient, which may also be used to more accurately the fluid dynamics of the blood flow for that particular patient.

In an embodiment, the extracting of the at least one vessel parameter form the intravascular image data comprises an extracting of a parameter indicating a bifurcation in the vasculature. In this context, a bifurcation in the vasculature may particularly refer to a bifurcation in the vessel of interest, i.e. a bifurcation of the vessel tree. The adapting of the physiological model comprises an including of the bifurcation into the fluid dynamics model.

The intravascular images comprised in the intravascular image data may further show small bifurcations which may not be resolved in the at least one diagnostic image. Bifurcations in the vessels have an influence on the blood flow dynamics. Thus, in order to provide an accurate fluid dynamics model, these bifurcations need to be considered as well by adjusting the boundary conditions in accordance with the additional bifurcations.

According to a further aspect, a method for modeling coronary physiology is provided. The method comprises the steps of receiving at least one diagnostic image of a vasculature of a patient, and intravascular image data obtained in-situ from a vessel of said vasculature and generating, on the basis of the at least one diagnostic image, a physiological model of the patient, the physiological model comprising a geometric model of the vessel of the vasculature. The method further comprises the step of extracting at least one vessel parameter of the vessel of the vasculature from the intravascular image data and of adapting the physiological model on the basis of the at least one vessel parameter.

Herein, the co-registration of at least one non-invasively obtained diagnostic image, in particular of a single 2D X-ray angiography image, together with invasively obtained intravascular image data is used to obtain patient-specific vessel parameters to be used in the physiological model.

These patient-specific vessel parameters in particular include hemodynamic parameters, such as vessel wall elasticity, vessel wall composition, blood viscosity, or the like. These hemodynamic parameters influence the fluid dynamic of the blood flow for each patient. Accordingly, in order to provide a more accurate fluid dynamics model for simulating blood flow, these patient-specific hemodynamic parameters should be included in the calculation of the fluid dynamics model. This is done by including these hemodynamic parameters as determined from the intravascular image data as patient-specific boundary conditions into the calculation, thereby replacing the previously used generalized boundary conditions. As a result, the calculated fluid dynamics model more accurately represents the fluid dynamics of the blood flow through the vessels for that particular patient.

In a further aspect, a computer program for controlling an apparatus according to the invention is provided, which, when executed by a processing unit, is adapted to perform the method steps according to the invention. In an even further aspect, a computer-readable medium is provided having stored thereon the above-cited computer program.

It shall be understood that the apparatus of claim 1, the method of claim 13, the computer program of claim 14 and the computer-readable medium of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically illustrates an apparatus for modeling coronary physiology according to an embodiment.
Fig. 2 represents a flow chart for a method for modeling coronary physiology according to an embodiment.
Fig. 3A shows an illustration of a combination of an angiographic image with intravascular ultrasound image data.
Fig. 3B represents an adaption of the geometric model in the physiological model using cross sectional vessel lumen for a vessel segment as obtained from the intravascular ultrasound image data.
Fig. 4 schematically illustrates a lumped fluid dynamics model in which hemodynamic parameters retrieved from intravascular ultrasound data are provided as a further input to the lumped fluid dynamics model.

### DETAILED DESCRIPTION OF EMBODIMENTS

The illustration in the drawings is schematically. In different drawings, similar or identical elements are provided with the same reference numerals.

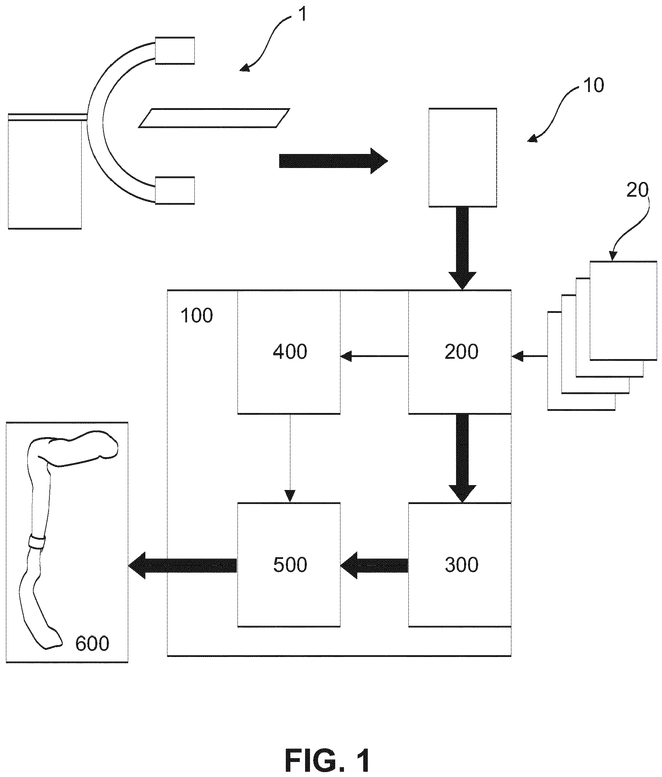

Fig. 1 represents schematically an exemplary embodiment of an apparatus 100 for modeling coronary physiology. In the embodiment, a C-arm system 1 for acquiring X-ray angiography projection data is used. That is the diagnostic image 10 is obtained by of X-ray angiography using a contrast agent. The diagnostic image 10 is a 2D X-ray angiography image. The intravascular imaging is performed using intravascular ultrasound imaging and the intravascular imaging data corresponds to a plurality of intravascular ultrasound images 20, which have been obtained by slowly pulling a catheter including a UV probe through the vessel.
The apparatus 100 comprises an input unit 200 for receiving the single 2D X-ray angiography image 10 and the plurality of intravascular ultrasound images 20 which have been obtained in co-registration for a particular patient. In the exemplary embodiment of Fig. 1, the input unit 200 further receives respective tracking information with the plurality of intravascular ultrasound images 20. This tracking information allows to determine the position of the vessel of interest at which the intravascular ultrasound image 20 has been collected. Accordingly, the tracking information enables a correlation of each intravascular ultrasound image 20 with a respective intravascular imaging position.

The input unit 200 provides the X-ray angiography image 10 to modeling unit 300. Modeling unit 300 is adapted to receive the X-ray angiography 10 and to perform a vessel segmentation of the vessel of interest in the imaged vasculature in the X-ray angiography image 10 to generate physiological model of the vessel including a respective geometric model. Thus, the modeling unit 300 is adapted to provide a modeled representation of the vessel geometry from the image information provided by the X-ray angiography image 10.

The input unit 200 further provides the plurality of intravascular ultrasound images 20 along with the respective tracking information to the extraction unit 400. The extraction unit 400 is adapted to correlate each of the intravascular ultrasound images 20 to a respective intravascular imaging position. Further, the extraction unit is configured to extract from each one of the plurality of intravascular ultrasound images 20 at least one vessel parameter.

In the exemplary embodiment of Fig. 1, extraction unit extracts cross sectional vessel lumen at the particular intravascular imaging position. Further, the extraction unit 400 extracts a value for the blood viscosity of the respective patient from the speckle concentration in the intravascular ultrasound image data. The extraction unit 400 provides the extracted cross sectional vessel lumen for each intravascular imaging position together with the tracking information as well as the patient-specific value for the blood viscosity to the adaption unit 500.

Adaption unit 500 is adapted to receive the at least one vessel parameter. Thus, in the exemplary embodiment of Fig. 1, adaption unit 500 receives the plurality of cross sectional vessel lumen for each intravascular imaging position and the determined value for the blood viscosity. Adaption unit 500 is configured to correlate each one of the plurality of intravascular imaging positions for which the extraction unit 400 has provided a respective cross sectional vessel lumen to a respective vessel position in the generated model based on the tracking information.

In the example according to Fig. 1, the adaption unit 500 then uses this correlated cross sectional vessel lumen information to adapt the geometric model comprised in the physiological model by adjusting the modeled cross sectional vessel lumen to the extracted cross sectional vessel lumen at the respective vessel positions. The cross sectional vessel lumen for each position that had previously been estimated from the 2D angiographic image is therefore replaced by a cross sectional vessel lumen that has been determined using intravascular ultrasound.

Further, the adaption unit 500 is adapted to use the value for the blood viscosity provided as part of the intravascular ultrasound data to obtain a patient-specific boundary condition for the fluid dynamics model in the physiological model. The adaption unit 500 then uses the thus obtained patient-specific boundary condition for an adaption of the fluid dynamics model, i.e. by calculating the fluid dynamics using the patient-specific boundary condition, such as to more accurately determine blood flow through the vessel of interest.

Thus, the physiological model is adapted to a patient with respect to the vessel geometry as well as the blood flow simulations. Upon finalizing the adaption, the adaption unit 500 outputs a patient-specific physiological model to display unit 600. The patient-specific physiological model that is displayed allows for a more accurate determination of FF/iFR.

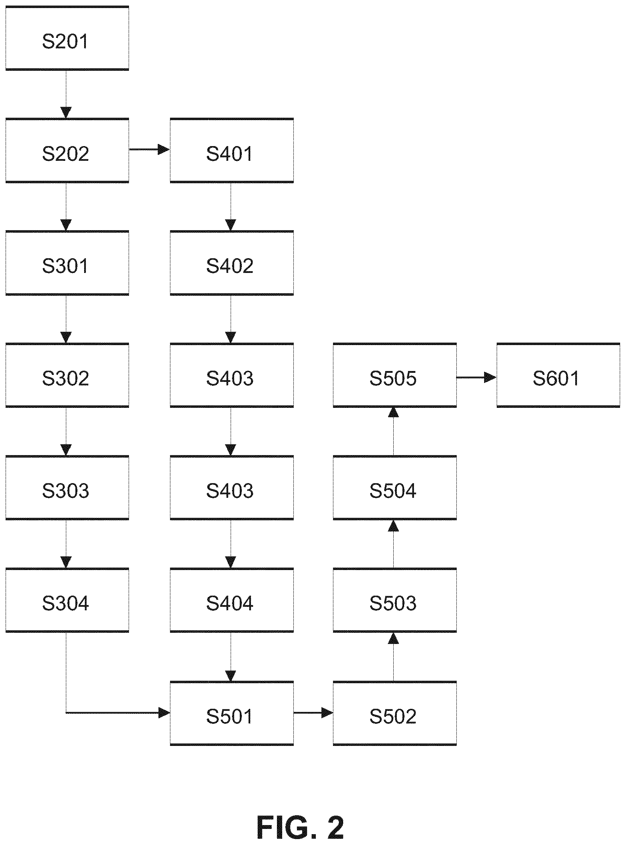

Fig. 2 illustrates a flow chart for a method for modeling coronary physiology as performed by the apparatus according to Fig.1. .In step S201, the 2D X-ray angiography image 10 of a vasculature of a patient is received. Further, the plurality of intravascular ultrasound images 20 along with respective tracking information is received. In step S202, the input unit 200 provides the 2D X-ray angiography image 10 to the modeling unit 300. Further, the input unit 200 provides the plurality of intravascular ultrasound images 20 along with the respective tracking information to the extraction unit 400.

In step S301, the 2D X-ray angiography image 10 is received at the modeling unit 300. The modeling unit 300 identifies a vessel of the vasculature and, in step S302, optionally performs a segmentation of a vessel of interest into a plurality of segments. In step S303, the modeling unit 300 then generates a physiological model including a geometric model of the vessel of interest and a fluid dynamics model representing the blood flow through said vessel of interest. Finally, in step S304, the modeling unit 300 provides the physiological model to the adaption unit 500.

In step S401, the plurality of intravascular images is received, along with the respective tracking information, at the extraction unit 400. In the exemplary embodiment of Fig. 2, the extraction unit 400, in step S402, correlates the intravascular imaging positions and the intravascular ultrasound images 20. Thus, in step S402, it may be determined which intravascular ultrasound image 20 has been obtained at which intravascular imaging position in the vessel. The thus correlated data is then used for further processing.

In step S403, the extraction unit 400 extracts at least one vessel parameter from the intravascular image data. In the specific example of Fig. 2, the cross sectional vessel lumen is extracted as a geometric parameter from each of the plurality of intravascular ultrasound images 20 in step S403. When this extraction is performed on the basis of the correlated intravascular ultrasound images, a cross sectional vessel lumen for each intravascular imaging position may be extracted by the extraction unit 400.

In step S404, the extraction unit 400, further extracts at least one vessel parameter that corresponds to a hemodynamic parameter from the intravascular image data. In the particular example of Fig. 2, the extraction unit 400 extracts a value for the blood viscosity for the patient from the speckles present in the intravascular ultrasound image data. In step S405, the plurality of the cross sectional vessel lumen for each intravascular imaging position, the tracking information provided from the input unit and the value for the blood viscosity specific to the patient are provided to the adaption unit 500.

In step S501, the adaption unit 500 receives the extracted vessel parameters. Thus, in the example according to Fig. 2, the adaption unit 500 receives a plurality of cross sectional vessel lumen for each intravascular image position as well as the value of the blood viscosity values. In step S502, the adaption unit 500 optionally correlates each one of the plurality of intravascular imaging positions to a respective vessel position in the geometric model of the vessel based on the tracking information. In step S503, the adaption unit 500 adapts the cross sectional vessel lumen at the respective vessel positions in the geometric model.

In step S504, the adaption unit 500 may use the hemodynamic parameter, in this exemplary embodiment the value of the blood viscosity for the respective patient, to define a patient-specific boundary condition to be used in the fluid dynamics model for the blood flow through the patient's vessel. In step S505, the adaption unit 500 applies this patient-specific boundary condition to adapt the fluid dynamics model. The adaption unit 500 in particular uses the patient-specific boundary condition perform a blood viscosity correction of the fluid dynamics calculation previously performed based on the generalized boundary conditions.

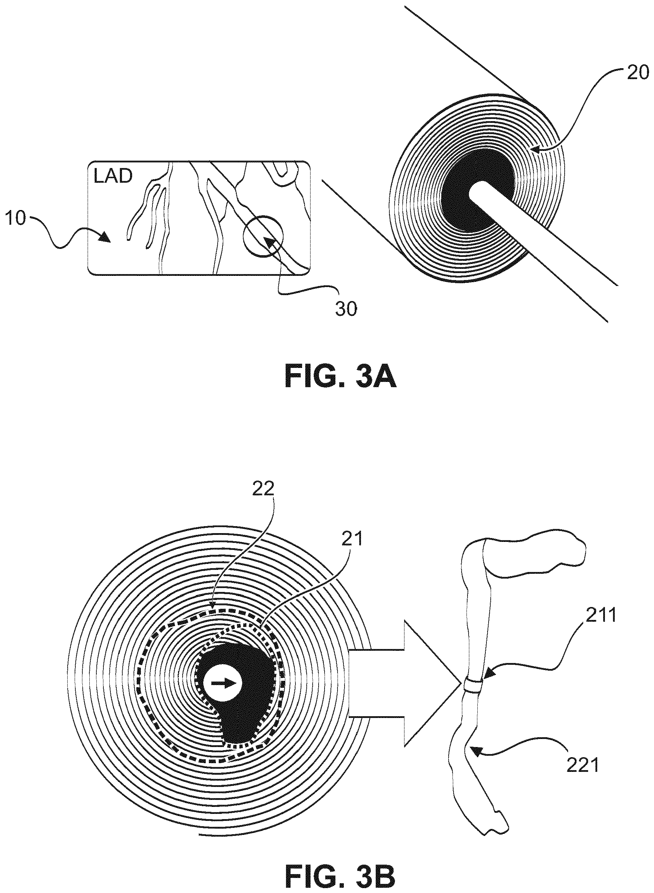

Optionally, in step S601, display unit 600 receives the patient-specific physiological model including the patient-specific geometric model as well as the patient-specific fluid dynamics model. In the exemplary embodiment of Fig. 2, the physiological model, that is a graphical representation thereof, is displayed for further evaluation. Fig. 3A illustrates a combination of a 2D X-ray angiography image 10 and an intravascular ultrasound image 20. The vessel of interest 30 in the 2D X-ray angiography image 10 is highlighted by a circle. As may be appreciated from Fig. 3A, the borders of the vessel of interest 30 are clearly defined in the 2D X-ray angiography image 10. Thus, the geometric shape of the vessel of interest 30 in two dimensions may be directly derived from the 2D X-ray angiography image 10. The intravascular ultrasound image 20 has been obtained in-situ inside the vessel. Accordingly, the intravascular ultrasound image 20 may be used to obtain information about the cross sectional vessel lumen, i.e. the information about the third dimension that is not available from the angiographic image.

To that end, Fig. 3B shows a segmentation of a vessel to derive the respective cross sectional vessel lumens along said vessel from the intravascular ultrasound image 20. The dotted line 21 hereby represents the cross sectional vessel lumen for a first segment of the vessel at a first intravascular imaging position and the dashed line 22 represents the cross sectional vessel lumen for a second segment of the vessel at a second intravascular imaging position.

From these segments, the respective cross sectional vessel lumen are extracted and input into the adaption unit 500 to adapt the geometric model accordingly. Thus, the initial cross sectional vessel lumen at a vessel position 211 corresponding to the first intravascular imaging position is adapted to the cross section vessel lumen 21. Likewise, the initial cross sectional vessel lumen at a vessel position 221 corresponding to the second intravascular imaging position is adapted to the cross section vessel lumen 22.

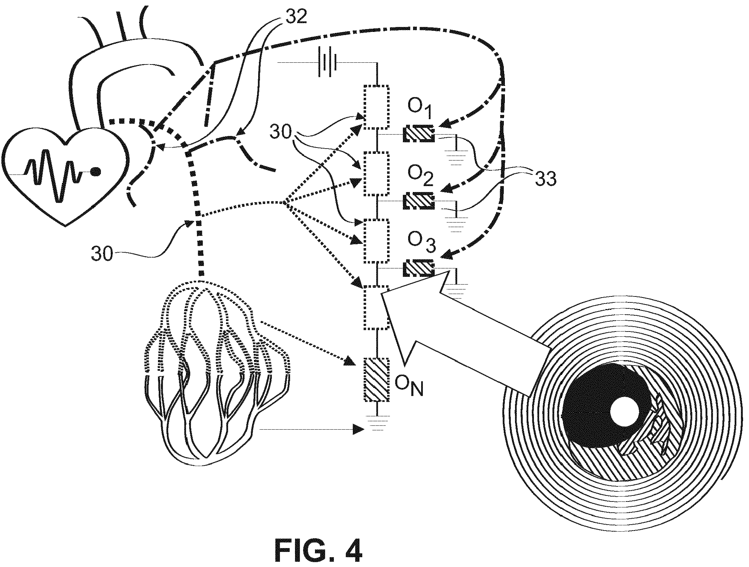

Fig. 4 illustrates a lumped fluid dynamics model in which the hemodynamic parameters retrieved from the intravascular ultrasound data are provided as a further input. The local fluid vessel resistance of vessel 30 is represented by a plurality of resistor elements 31, each having a particular resistance. The outlets 32 of vessel 30 at the different positions are modeled as respective resistances 33 between the resistor elements 31.

The intravascular ultrasound images 20 allow retrieval of certain information of about the properties of the vessel relating to the fluid dynamics within the vessel. In the example of Fig. 4, the vessel wall composition is derived from the intravascular ultrasound images. The vessel wall composition relates to fluid friction, and, thus, to the fluid vessel resistance. Accordingly, the values for the resistor elements 31 as boundary conditions of the lumped fluid dynamics model have to be adjusted in accordance with the vessel wall composition determined by the intravascular ultrasound images 20. By means of this adjustment, the fluid dynamics model may be adapted to a patient-specific fluid dynamics model.

Although in above described embodiments, the diagnostic images have been obtained using X-ray angiography, it shall be understood that in other embodiments, the diagnostic images may be retrieved by other imaging methods, such as helical computed tomography or sequential computed tomography, magnetic resonance imaging, ultrasound imaging, or the like.

Likewise, it may be understood that the intravascular image data may be obtained by other intravascular imaging methods other than intravascular ultrasound and/or optical coherence tomography, such as endoscopy or the like.

It may also be understood that the above approach of co-registering the information from two imaging modalities with one another to improve a physiological model of a patient's vessel may also be applied to the co-registration of two non-invasive imaging modalities or two-invasive imaging modalities as long as they may provide corresponding information.

Further, while in the above embodiments, the modeling has been performed on the coronary physiology, in other embodiments, the modeling may likewise be performed on other image-derived physiologies of the human body. As an example, the approach may be applied to model the peripheral arteries in the human body.

It may further be understood that while in the above-embodiments, the cross sectional vessel lumen and the inner shape of the vessel have been derived as geometric parameters from the intravascular images, other geometric parameters may likewise be derived such as the diameter at a particular position along a pre-defined direction of the vessel or a length of a narrowing of the vessel or the like.

Although in the above described embodiments the hemodynamic parameters derived from the intravascular imaging data relate to vessel wall elasticity, blood viscosity and vessel wall friction, a skilled person will understand that the above described methods may likewise be used to obtain further information such as local flow velocity.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the correlation of the intravascular images to their intravascular imaging positions, the generating of a physiological model, the extraction of the parameters or the adaption of the physiological model et cetera performed by one or several units or devices can be performed by any other number of units or devices. These procedures in accordance with the invention can hereby be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an apparatus for modeling coronary physiology. The apparatus comprises an input unit configured to receive at least one diagnostic image of a vasculature of a patient, and intravascular image data obtained in-situ from a vessel of the vasculature. The apparatus also comprises a modeling unit configured to generate, on the basis of the at least one diagnostic image, a physiological model of the patient, the physiological model comprising a geometric model of the vessel of the vasculature, an extraction unit configured to extract at least one vessel parameter of the vessel of the vasculature from the intravascular image data and an adaption unit configured to adapt the physiological model on the basis of the at least one vessel parameter.

## Claims

1. An apparatus for modeling coronary physiology, comprising
an input unit configured to receiving
at least one diagnostic image of a vasculature of a patient, and
intravascular image data obtained in-situ from a vessel of the vasculature;
a modeling unit configured to generate, on the basis of the at least one diagnostic image, a physiological model of the patient, the physiological model comprising a geometric model of the vessel of the vasculature;
an extraction unit configured to extract at least one vessel parameter of the vessel of the vasculature from the intravascular image data; and
an adaption unit configured to adapt the physiological model on the basis of the at least one vessel parameter.

2. The apparatus according to claim 1, wherein
the at least one diagnostic image is obtained using X-ray angiography.

3. The apparatus according to claim 1, wherein
the intravascular image data is obtained using intravascular ultrasound (IVUS) and/or optical coherence tomography (OCT).

4. The apparatus according to claim 1, wherein
the intravascular image data comprises a time-series of intravascular images obtained during a cardiac cycle of the patient.

5. The apparatus according to claim 1, wherein
the generating of the physiological model comprises a segmenting of the vessel of the vasculature; and wherein
the geometric model is generated for at least one segment of the vessel.

6. The apparatus according to claim 1, wherein
the intravascular image data comprises a plurality of intravascular images, wherein each one of the plurality of intravascular image has been obtained at one of a plurality of different intravascular imaging positions along the vessel of the vasculature.

7. The apparatus according to claim 6, wherein
the input unit is further configured to receive tracking information for correlating each of the plurality of intravascular imaging positions to a respective vessel position in the geometric model.

8. The apparatus according to claim 7, wherein
the extracting of at least one vessel parameter from the intravascular image data comprises an extracting of a geometric parameter from each one of the plurality of intravascular images obtained at each one of the plurality of the different intravascular imaging positions; and
the adapting of the physiological model comprises an adapting of the geometric model of the vessel at the respective vessel position.

9. The apparatus according to claim 1, wherein
the apparatus further comprises a display unit configured to display a graphical representation of the physiological model; wherein
the adapting of the physiological model is performed in real-time during obtaining of the intravascular image data; and
wherein the display unit is further configured to update the graphical representation based on the adapting of the physiological model.

10. The apparatus according to claim 1, wherein
the physiological model further comprises a fluid dynamics model for a blood flow through the geometric model of the vessel; and wherein
the extracting of at least one vessel parameter from the intravascular image data comprises an extracting of at least one hemodynamic parameter, wherein
the adapting of the physiological model comprises an adapting of the fluid dynamics model on the basis of the at least one hemodynamic parameter.

11. The apparatus according to claim 10, wherein
the adapting of the physiological model comprises adjusting at least one patient-specific boundary condition for the fluid dynamics model on the basis of the at least one hemodynamic parameter.

12. The apparatus according to claim 10, wherein
the extracting of the at least one vessel parameter from the intravascular image data comprises an extracting of a parameter indicating a bifurcation in the vasculature; and
the adapting of the physiological model comprises an including of the bifurcation into the fluid dynamics model.

13. A method for modeling coronary physiology, the method comprising
receiving at least one diagnostic image of a vasculature of a patient, and intravascular image data obtained in-situ from a vessel of said vasculature;
generating, on the basis of the at least one diagnostic image, a physiological model of the patient, the physiological model comprising a geometric model of the vessel of the vasculature;
extracting at least one vessel parameter of the vessel of the vasculature from the intravascular image data; and
adapting the physiological model on the basis of the at least one vessel parameter.

14. A computer program for controlling an apparatus according to anyone of claims 1 to 11, which, when executed by a processing unit, is adapted to perform the method according to claim 13.

15. A computer-readable medium having stored there on the computer program according to claim 14.
